(19) Europäisches Patentamt
European Patent Office
Office européen des brevets

(11) Numéro de publication: **0 127 529**
**A2**

(12) # DEMANDE DE BREVET EUROPEEN

(21) Numéro de dépôt: 84401032.2

(22) Date de dépôt: 18.05.84

(51) Int. Cl.³: **C 07 D 451/04**
C 07 D 451/14, A 61 K 31/46
A 61 K 31/445

(30) Priorité: 20.05.83 FR 8308350

(43) Date de publication de la demande: 05.12.84 Bulletin 84/49

(84) Etats contractants désignés: BE CH DE FR GB IT LI LU NL SE

(71) Demandeur: DELALANDE S.A.
32, rue Henri Regnault
F-92400 Courbevoie(FR)

(72) Inventeur: Dostert, Philippe
10, place des Vosges
F-75004 Paris(FR)

(72) Inventeur: Imbert, Thierry
8, Hameau de la Lévrière
F-78590 Noisy Le Roi(FR)

(72) Inventeur: Bucher, Bernard
23, Boulevard de la République
F-92430 Marnes La Coquette(FR)

(74) Mandataire: Kedinger, Jean-Paul et al,
c/o Cabinet Malemont 42, avenue du Président Wilson
F-75116 Paris(FR)

(54) **N-oxydes de dérivés aminocycliques, leur procédé de préparation et leur application en thérapeutique.**

(57) N-oxydes de dérivés aminocycliques, répondant à la formule:

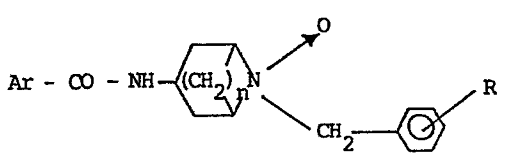

(I)

dans laquelle n = 2 ou 3; R = H, halogène(s), alkyle en $C_1$-$C_4$, alkyloxy en $C_1$-$C_4$, CN ou $CF_3$; et Ar = groupe pyrimidinique substitué ou phényle substitué.

Ces composés sont utiles comme médicaments notamment en tant que neuroleptiques.

EP 0 127 529 A2

N-oxydes de dérivés aminocycliques, leur procédé de préparation et leur application en thérapeutique

La présente invention a pour objet de nouveaux N-oxydes de dérivés aminocycliques, leur procédé de préparation et leur application en thérapeutique.

Plus précisément, les nouveaux composés selon l'invention répondent à la formule :

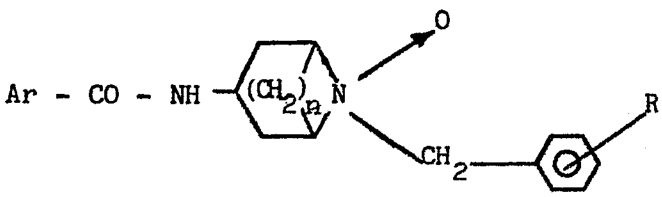

(I)

dans laquelle R représente un atome d'hydrogène, un ou deux atomes d'halogène ou un groupe alkyle en $C_1$-$C_4$, alkyloxy en $C_1$-$C_4$, cyano ou trifluorométhyle, n prend la valeur 2 ou 3 et Ar représente :

a) un groupe pyrimidinique de formule :

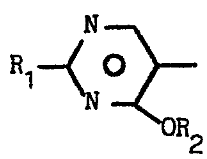

où $R_1$ = H, alkyle en $C_1$-$C_4$, alkoxy en $C_1$-$C_4$, $NH_2$, NH-alkyl en $C_1$-$C_4$, N(alkyl en $C_1$-$C_4$)$_2$, pyrrolidino, pipéridino ou morpholino et $R_2$ = alkyle en $C_1$-$C_4$ ;

b) un groupe aromatique de formule :

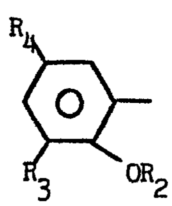

ou

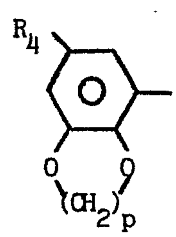

où p = 1 ou 2,

$R_2$ a les mêmes significations que précédemment,

$R_3$ = OH, alkyloxy en $C_1$-$C_4$ ou alkyle en $C_1$-$C_4$ et

$R_4$ = halogène, OH, alkyloxy en $C_1$-$C_4$, alkylsulfonyle en $C_1$-$C_4$,

$NO_2$, CN, CHO, (alkyl en $C_1$-$C_4$) carbonyle, $NH_2$, NH-alkyl en $C_1$-$C_4$, N(alkyl en $C_1$-$C_4$)$_2$, pyrrolidino, morpholino, pipéridino, $SO_2NH_2$, $SO_2$ N(alkyl en $C_1$-$C_4$)$_2$, pyrrolidinosulfonyle, pipéridinosulfonyle ou morpholinosulfonyle,

$R_3$ pouvant en outre représenter un groupe $NH_2$ ou NH-alkyl en $C_1$-$C_4$ quand $R_4$ = halogène et un atome d'hydrogène quand $R_4$ représente un groupe OH ou alkyloxy en $C_1$-$C_4$ ;

c) un groupe aromatique de structure :

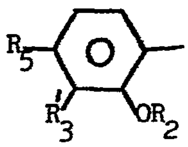

ou

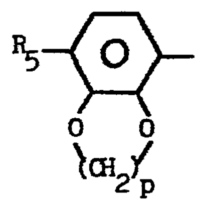

où $R_2$ et p ont les mêmes significations que précédemment, $R'_3$ = OH, alkyloxy en $C_1$-$C_4$ ou alkyle en $C_1$-$C_4$ et $R_5$ = $NH_2$, NH-alkyl en $C_1$-$C_4$, N(alkyl en $C_1$-$C_4$)$_2$, pyrrolidino, pipéridino, morpholino, halogène ou alkyloxy en $C_1$-$C_4$, $R'_3$ pouvant en outre représenter un atome d'hydrogène quand $R_5$ représente un groupe alkyloxy en $C_1$-$C_4$ ; ou

d) un groupe aromatique de structure :

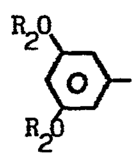

où $R_2$ a la même signification que précédemment.

Il est à noter que les composés selon l'invention :

- se présentent sous la forme axiale ($I_a$) :

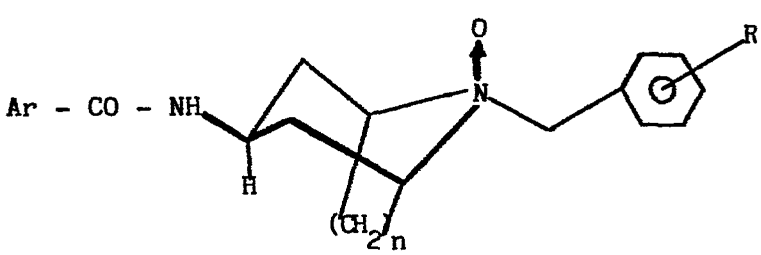

(Ia)

où la chaîne ArCONH est en position équatoriale et l'atome d'oxygène du fragment >N → O est en position axiale ou sous la forme équatoriale (Ib) :

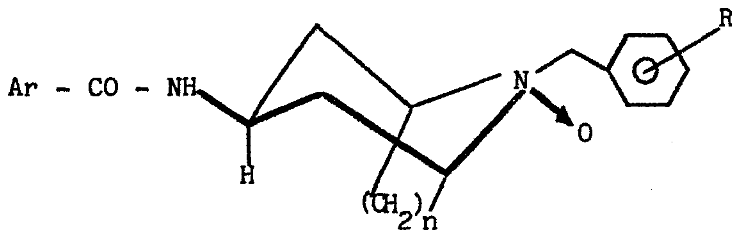

(Ib)

où la chaîne ArCONH et l'atome d'oxygène du fragment >N → O sont tous deux en position équatoriale ; ou

- sont constitués par un mélange de la forme axiale (Ia) et de la forme équatoriale (Ib).

Les composés de formule (I) selon l'invention peuvent être obtenus par oxydation des composés de formule :

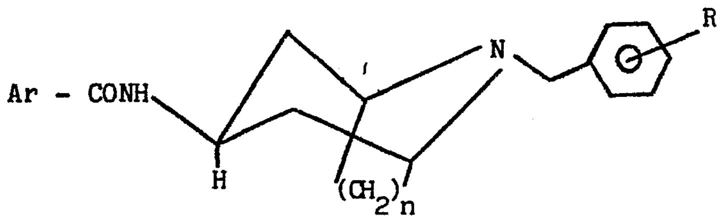

(II)

dans laquelle la chaîne ArCONH est en position équatoriale, n = 2 ou 3 et R et Ar ont la même signification que dans la formule (I), éventuellement suivie de la séparation des formes (Ia) et (Ib).

Avantageusement, on réalise l'oxydation par mise en oeuvre d'un peracide tel que l'acide métachloroperbenzoïque ou d'un peroxyde tel que l'eau oxygénée ; quand on utilise l'acide métachloroperbenzoïque, on opère de préférence dans un solvant aprotique tel que le chlorure de méthylène et à basse température telle que 0° C et quand on utilise l'eau oxygénée, on opère de préférence au reflux dans un solvant aprotique tel que l'acétonitrile.

La séparation des formes (Ia) et (Ib) peut être notamment réalisée par chromatographie sur colonne, de préférence par chromatographie liquide sur colonne de silice, à haute ou moyenne pression, les formes séparées étant identifiées par leurs propriétés spectrales, notamment à l'aide des spectres de R.M.N.

Les composés de formule (II) sont quant à eux obtenus conformément aux protocoles opératoires décrits dans les brevets français 2 446 823, 2 476 088 et 2 493 848.

Les préparations suivantes sont données à titre d'exemple non limitatif pour illustrer l'invention.

<u>N-oxyde axial du (diméthoxy-2,3) benzoylamino-3 N-benzyl aza-8 bicyclo [3.2,1] octane [(Ia), numéro de code 1] et N-oxyde équatorial du (diméthoxy-2,3) benzoylamino-3 N-benzyl aza-8 bicyclo[3.2,1] octane [(Ib) , numéro de code 2]</u>

(1) A une solution de 6,5 g de β-(diméthoxy-2,3) benzoylamiro-3 N-benzyl aza-8 bicyclo [3.2.1] octane (II) dans 150 ml de chlorure de méthylène refroidie à 0° C, on ajoute 3,6 g d'acide métachloroperbenzoïque ; on laisse 10 minutes en contact, puis on lave à l'aide de NaOH 1 N, décante, sèche la phase organique sur sulfate de sodium, filtre et évapore le filtrat. L'huile brute obtenue est chromatographiée sur une colonne de silice (chromatographie liquide à moyenne pression). Par élution à l'aide du mélange chlorure de méthylène 95 % - méthanol 5 %, on obtient (a) 4,5 g (66 %) du N-oxyde axial (Ia) (numéro de code 1) que l'on recristallise dans le pentane:

- Point de fusion : 210° C
- Formule brute : $C_{23}H_{28}N_2O_4$
- Poids moléculaire : 396,47
- <u>Spectre de R.M.N.</u> (à 250 MHz ; $CD_3OD$) δ ppm =
  - entre 7,05 et 7,65, m, (8 protons benzéniques et CON<u>H</u>)
  - 4,5, m, proton axial en 3
  - 4,42, s, $-C\underline{H_2}$ benzylique
  - 3,82 et 3,88, s, 2 $C\underline{H_3}O$
  - 3,53, s, 2 protons tropaniques en 1 et 5
  - 2,5, m, protons tropaniques axiaux en 2 et 4 et 2 protons tropaniques en 6 et 7
  - 2,25, m, 2 protons tropaniques en 6 et 7
  - 1,92, m, 2 protons tropaniques équatoriaux en 2 et 4

puis (b) 1,2 g (17 %) du N-oxyde équatorial (Ib) (numéro de code 2) que l'on recristallise dans l'éther éthylique:

. Point de fusion : 235° C

. Formule brute : $C_{23}H_{28}N_2O_4$

. Poids moléculaire : 396,47

. Spectre de R.M.N. (à 250 MHz ; $CD_3OD$) $\delta$ ppm =

. 7,83, m, CON<u>H</u>

. entre 7,12 et 7,45, m, 8 protons benzéniques

. 4,8, s, $C\underline{H}_2$ benzylique

. 4,5, m, proton tropanique axial en 3

. 3,88 et 3,91, s, 2 $C\underline{H}_3O$

. 3,6, s, 2 protons tropaniques en 1 et 5

. entre 2,5 et 2,7, m, 2 protons tropaniques axiaux en 2 et 4 et 2 protons tropaniques en 6 et 7

. 2,13, m, 2 protons tropaniques équatoriaux en 2 et 4

. 1,96, m, 2 protons tropaniques en 6 et 7

(2) A une solution à 20° C de 26 g de $\beta$-(diméthoxy-2,3) benzoyl amino-3 N-benzyl aza-8 bicyclo [3.2.1] octane (II) dans 300 ml d'acétonitrile, on ajoute lentement 150 ml d'eau oxygénée à 120 volumes. Puis on porte le mélange 30 minutes au reflux. Après refroidissement on ajoute du palladium sur charbon à 5 %, humide, pour détruire l'excès d'eau oxygénée. Puis on filtre, évapore le filtrat et chromatographie le résidu sur colonne de silice (chromatographie liquide à moyenne pression). Par élution par le mélange chlorure de méthylène 95 % - méthanol 5 %, on obtient 9 g (33 %) de N-oxyde axial [(Ia), numéro de code 1], puis par élution par le mélange chlorure de méthylène 85 % - méthanol 15 %, on obtient 9 g (33 %) de N-oxyde équatorial [(Ib), numéro de code 2] identiques pour les spectres RMN aux produits obtenus par la méthode (1) .

Nous noterons ici que l'attribution axiale et équatoriale des N-oxydes (Ia) et (Ib) a été faite selon l'étude RMN des N-oxydes tropaniques décrite dans Helvetica Chimica Acta. <u>55</u>, 637, (1972).

Par les mêmes procédés (1) ou (2) précédemment décrits, mais à partir des réactifs correspondants, on obtient les composés de formule (I) de numéros de code 3 à 8, 13 à 16, 18 et 19 répertoriés dans le tableau I suivant.

TABLEAU I

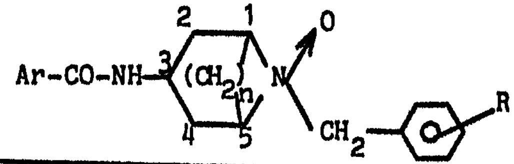

(I)

| Numéro de Code | Ar- | n | R | Forme | Formule brute | Poids molé-culaire | Point de fusion (°C) | ANALYSE ELEMENTAIRE ET/OU SPECTRE DE RMN |
|---|---|---|---|---|---|---|---|---|
| 1 | 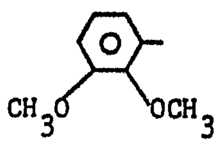 | 2 | H | (Ia) | $C_{23}H_{28}N_2O_4$ | 396,47 | 210 | RMN (250 MHz, $CD_3OD$), δ ppm =<br>. entre 7,05 et 7,65, m (8 H benzéniques et CONH)<br>. 4,5,m (proton axial en 3)<br>. 4,42,s (-$CH_2$-Ø)<br>. 3,82 et 3,88, s (2 $CH_3O$)<br>. 3,53, s (2 H en 1 et 5)<br>. 2,5, m (2 H axiaux en 2 et 4 + 2 H tropaniques en 6 et 7)<br>. 1,92, m (2 H équatoriaux en 2 et 4)<br>. 2,25, m (2 H tropaniques en 6 et 7) |
| 2 | 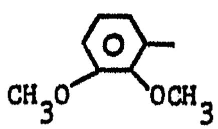 | " | " | (Ib) | " | " | 235 | RMN (250 MHz, $CD_3OD$), δ ppm =<br>. 7,83, m (CONH)<br>. entre 7,12 et 7,45, m (8 H benzéniques)<br>. 4, 8, s ($CH_2$-Ø)<br>. 4,5, m (H en 3)<br>. 3,88 et 3,91, s (2 $CH_3O$)<br>. 3,6, s (2 H en 1 et 5)<br>. entre 2,5 et 2,7, m (2 H axiaux en 2 et 4 et 2 H tropaniques en 6 et 7)<br>. 2,13, m (2 H équatoriaux en 2 et 4)<br>. 1,96,m (2 H tropaniques en 6 et 7) |

**TABLEAU I** (suite)

| Numéro de Code | Ar- | n | R | Forme | Formule brute | Poids moléculaire | Point de fusion (°C) | ANALYSE ELEMENTAIRE ET/OU SPECTRE DE RMN |
|---|---|---|---|---|---|---|---|---|
| 3 | 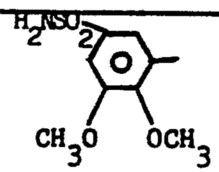 | 2 | H | (Ia) | $C_{23}H_{29}N_3O_6S$ + 1,7 % $H_2O$ | 483,78 | 257 | RMN (80 MHz, DMSO), δ ppm =<br>. 8,3, d (CONH)<br>. 7,2 à 7,8, m ($SO_2NH_2$ et 7 H benzéniques)<br>. 4,25, s ($CH_2$ Ø)<br>. 4,2, m (H en 3)<br>. 3,8 et 3,9, s (2 $CH_3O$)<br>. 3,2, m (H en 1 et 5)<br>. 1,6 à 2,35, m (8 H : 4 H en 2 et 4 et 4 H en 6 et 7)<br><br>\| \| C \| H \| N \|<br>Cal. % \| 57,10 \| 6,24 \| 8,69<br>Tr. % \| 57,02 \| 6,27 \| 8,20 |
| 4 | 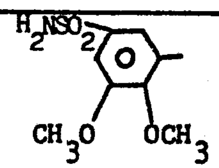 | " | " | (Ib) | $C_{23}H_{29}N_3O_6S$ + 2 % $H_2O$ | 485,26 | 220 | RMN (80 MHz, DMSO), δ ppm =<br>. 8,4, d (CONH)<br>. 7,2 à 7,8, m ($SO_2NH_2$ + 7 H benzéniques)<br>. 4,6, s (-$CH_2$-Ø)<br>. 4,4, m (H en 3)<br>. 3,8 et 3,9, s (2 $CH_3O$)<br>. 3,35, m (H en 1 et 5)<br>. 1,5 à 2,6, m (8 H tropaniques en 2,4,6,7)<br><br>\| \| C \| H \| N \|<br>Cal. % \| 56,93 \| 6,25 \| 8,66<br>Tr. % \| 56,95 \| 6,31 \| 8,55 |

TABLEAU I (suite)

| Numéro de Code | Ar- | n | R | Forme | Formule brute | Poids moléculaire | Point de fusion (°C) | ANALYSE ELEMENTAIRE ET/OU SPECTRE DE RMN |
|---|---|---|---|---|---|---|---|---|
| 5 | 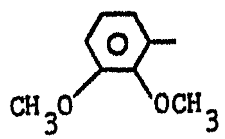 | 3 | H | (Ia) | $C_{24}H_{30}N_2O_4$ + 2,5 % $H_2O$ | 421,02 | 188 | RMN (80 MHz, DMSO), δ ppm =<br>. 8,05, d (CONH)<br>. 7,1 à 7,8, m (8 H benzéniques)<br>. 4,45, s ($-CH_2-Ø$)<br>. 4,55, m (H en 3)<br>. 3,75 et 3,85, s (2 $CH_3O$)<br>. 3,0, m (H en 1 et 5)<br>. 1,5 à 2,85, m (10 H en 2,4,6,7,8)<br>— C / H / N —<br>Cal. %: 68,46 / 7,46 / 6,65<br>Tr. %: 68,46 / 7,72 / 6,32 |
| 6 | " | " | " | (Ib) | $C_{24}H_{30}N_2O_4$ + 1,2 % $H_2O$ | 415,48 | " | RMN (80 MHz, DMSO) δ ppm =<br>. 8,25, d (CONH)<br>. 7 à 7,85, m (8 H benzéniques)<br>. 4,6, m (H en 3)<br>. 4,6, s ($CH_2$ -Ø)<br>. 3,75 et 3,8, s (2 $CH_3O$)<br>. 2,95, m (H en 1 et 5)<br>. 1,3 à 2,6, m (10 H en 2,4,6,7,8)<br>— C / H / N —<br>Cal. %: 69,38 / 7,41 / 6,74<br>Tr. %: 69,56 / 7,65 / 6,78 |

**TABLEAU I** (suite)

| Numéro de Code | Ar- | n | R | Forme | Formule brute | Poids molé-culaire | Point de fusion (°C) | ANALYSE ELEMENTAIRE ET/OU SPECTRE DE RMN |
|---|---|---|---|---|---|---|---|---|
| 7 | 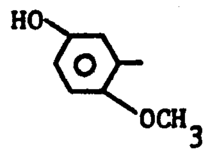 | 2 | H | (Ia) | $C_{22}H_{26}N_2O_4$ + 4,5 % $H_2O$ | 400,46 | 248 | RMN (80 MHz, DMSO), $\delta$ ppm =<br>. 9,5, m (OH)<br>. 7,95, d (CONH)<br>. 6,7 à 7,7, m (8 H benzéniques)<br>. 4,35, s ($-CH_2\emptyset$)<br>. 4,25, m (H en 3)<br>. 3,7, s ($CH_3O$)<br>. 3,15, m (H en 1 et 5)<br>. 1,5 à 2,7, m (8 H en 2,4,6 et 7)<br>    C   H   N<br>Cal. % 65,98   7,04   7,00<br>Tr. % 65,96   6,94   6,65 |
| 8 | " | " | " | (Ib) | $C_{22}H_{26}N_2O_4$ | 382,44 | 253 | RMN (80 MHz, DMSO), $\delta$ ppm =<br>. 8,2, d (CONH)<br>. 6,8 à 7,9, m (8 H benzéniques)<br>. 4,65, s ($-CH_2-\emptyset$)<br>. 4,35, m (H en 3)<br>. 3,7, s ($CH_3O$)<br>. 3,2, m (H en 1 et 5)<br>. 1,5 à 2,7, m (8 H en 2, 4, 6 et 7)<br>    C   H   N<br>Cal. % 69,09   6,85   7,33<br>Tr. % 69,09   6,81   7,10 |

TABLEAU I (suite)

| Numéro de Code | Ar- | n | R | Forme | Formule brute | Poids molé-culaire | Point de fusion (°C) | ANALYSE ELEMENTAIRE ET/OU SPECTRE DE RMN |
|---|---|---|---|---|---|---|---|---|
| 13 | 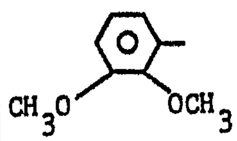 | 2 | 4-Br | (Ia) | $C_{23}H_{27}BrN_2O_4$ + 2 % $H_2O$ | 485,07 | 226 | RMN (80 MHz, DMSO), $\delta$ ppm =<br>. 8,1, d (CONH)<br>. 7 à 7,7, m (7 H benzéniques)<br>. 4,25 (s + m) : ($CH_2$Ø et H en 3)<br>. 4,75 et 4,8, s (2 $CH_3O$)<br>. 3,1, m (2 H en 1 et 5)<br>. 1,5 à 2,8, m (8 H en 2,4,6 et 7)<br><br>\| \| C \| H \| N \|<br>Cal. % \| 56,95 \| 5,83 \| 5,77<br>Tr. % \| 56,89 \| 5,95 \| 6,02 |
| 14 | " | " | " | (Ib) | $C_{23}H_{27}BrN_2O_4$ + 2 % $H_2O$ | 485,07 | 240 | RMN (80 MHz, DMSO), $\delta$ ppm =<br>. 8,3, d (CONH)<br>. 7 à 7,8, m (7 H benzéniques)<br>. 4,55, s ($CH_2$Ø)<br>. 4,3, m (H en 3)<br>. 3,7 et 3,8, s (2 $CH_3O$)<br>. 3,2, m (2 H en 1 et 5)<br>. 1,5 à 2,7, m (8 H en 2, 4, 6 et 7)<br><br>\| \| C \| H \| N \|<br>Cal. % \| 56,95 \| 5,83 \| 5,77<br>Tr. % \| 57,06 \| 5,78 \| 6,18 |

TABLEAU I (suite)

| Numéro de Code | Ar- | n | R | Forme | Formule brute | Poids molé-culaire | Point de fusion (°C) | ANALYSE ELEMENTAIRE ET/OU SPECTRE DE RMN |
|---|---|---|---|---|---|---|---|---|
| 15 | 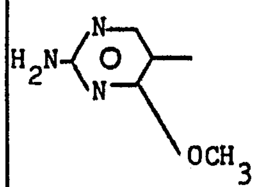 | 2 | 4-$CH_3$ | (Ia) | $C_{21}H_{27}N_5O_3$ + 2,2 % $H_2O$ | 406,41 | 255 | RMN (80 MHz, DMSO), δ ppm =<br>. 8,5, s (H pyrimidinique en 6)<br>. 7,0 à 7,65, m (4 H benzéniques + CONH)<br>. 4,25, (m + s) : (CH₂Ø et H en 3)<br>. 3,95, s ($CH_3O$)<br>. 3,1, m (2 H en 1 et 5)<br>. 2,35, s ($-CH_3$)<br>. 1,5 à 2,75, m (8 H en 2,4,6 et 7)<br><br>C / H / N<br>Cal. % 62,05 / 6,95 / 17,23<br>Tr. % 62,17 / 6,89 / 17,33 |
| 16 | " | " | " | (Ib) | $C_{21}H_{27}N_5O_3$ + 2,7 % $H_2O$ | 408,33 | 252 | RMN (80 MHz, DMSO), δ ppm =<br>. 8,5, s (H pyrimidinique en 6)<br>. 7,75, d (CONH)<br>. 7,1, à 7,75, m (4 H benzéniques)<br>. 4,6, s ($CH_2Ø$)<br>. 4,25, m (H en 3)<br>. 3,95, s ($CH_3O$)<br>. 3,3, m (2 H en 1 et 5)<br>. 2,35, s ($-CH_3$)<br>. 1,5 à 2,65, m (8 H en 2,4,6 et 7)<br><br>C / H / N<br>Cal. % 61,76 / 6,97 / 17,15<br>Tr. % 62,01 / 6,80 / 17,36 |

TABLEAU I (suite)

| Numéro de Code | Ar- | n | R | Forme | Formule brute | Poids moléculaire | Point de fusion (°C) | ANALYSE ELEMENTAIRE ET/OU SPECTRE DE RMN |
|---|---|---|---|---|---|---|---|---|
| 18 | 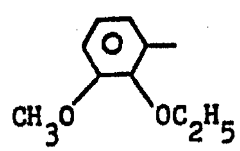 | 2 | H | (Ia) | $C_{24}H_{30}N_2O_4$ + 3,9 % $H_2O$ | 427,15 | 162 | RMN (80 MHz, DMSO), δ ppm = <br>. 8,15, d (CONH) <br>. 7,05 à 7,75, m (8 H benzéniques) <br>. 4,25, (m + s) : ($CH_2$-Ø + H en 3) <br>. 4, q et 1,25, t ($CH_3CH_2O$) <br>. 3,8, s ($CH_3O$) <br>. 3,1, m (2 H en 1 et 5) <br>. 1,5 à 2,85, m (8 H en 2,4,6 et 7) <br><br>C / H / N <br>Cal. % 67,48 / 7,52 / 6,55 <br>Tr. % 67,50 / 7,53 / 6,60 |
| 19 | 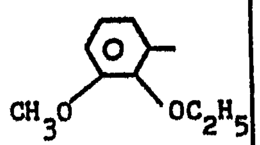 | 2 | H | (Ib) | $C_{24}H_{30}N_2O_4$ + 3,7 % $H_2O$ | 426,05 | 224 | RMN (80 MHz, DMSO), δ ppm = <br>. 8,3, d (CONH) <br>. 7, 05 à 7,85, m (8 H benzéniques) <br>. 4,65, s ($CH_2$Ø) <br>. 4,25, m (H en 3) <br>. 4,0, q et 1,3, t (O-$CH_2CH_3$) <br>. 3,85, s ($CH_3O$) <br>. 3,2, m (2 H en 1 et 5) <br>. 1,5 à 2,65, m (8 H en 2,4,6 et 7) <br><br>C / H / N <br>Cal. % 67,66 / 7,51 / 6,57 <br>Tr. % 67,46 / 7,43 / 6,51 |

Les composés selon l'invention ont été étudiés chez l'animal de laboratoire et ont montré une activité sur le système nerveux central, notamment une action neuroleptique.

Ces propriétés psychotropes ont été mises en évidence chez la souris, notamment par le test de l'antagonisme aux redressements à l'apomorphine réalisé d'après le protocole décrit par G. GOURET et Coll. dans J. Pharmacol. (Paris) (1973), 4, 341.

La toxicité aiguë des composés selon l'invention a également été étudiée chez la souris par voie intrapéritonéale. Le tableau II ci-après donne à titre d'exemple non limitatif les résultats obtenus avec certains des composés selon l'invention.

TABLEAU II

| Composé testé Numéro de code | Toxicité (souris) DL 50 (mg/kg/i.p.) | Antagonisme aux redressements à l'apomorphine (souris : DE 50 mg/kg/i.p.) |
|---|---|---|
| 1 | > 400 | 2,2 |
| 2 | 400 | 0,27 |
| 5 | 310 | 0,45 |
| 6 | 160 | 1,1 |
| 7 | > 400 | 2 |
| 8 | 330 | 1,5 |
| 13 | 325 | 0,9 |
| 14 | 280 | 0,22 |
| 15 | > 400 | 17 |
| 16 | > 400 | 1,2 |
| 18 | 150 | 1,1 |
| 19 | 280 | 0,5 |

Comme le montre le tableau ci-dessus, l'écart entre les doses toxiques et les doses actives permet l'emploi comme médicaments des composés (I), notamment dans le traitement des troubles du psychisme, notamment comme neuroleptiques.

La présente invention a donc également pour objet, à titre de médicaments, les composés de formule (I) pharmaceutiquement acceptables. L'invention s'étend aux compositions pharmaceutiques renfermant, à titre de principe actif, l'un au moins des médicaments définis ci-dessus, en association avec un véhicule pharmaceutiquement acceptable.

Ces compositions peuvent être administrées en particulier (a) par voie orale sous forme de comprimés, dragées ou gélules contenant chacun jusqu'à 300 mg de principe actif (jusqu'à 8 comprimés, dragées ou gélules par jour) ou sous forme de solution contenant de 0,1 à 1 % de principe actif (jusqu'à 60 gouttes, une à trois fois par jour) ou (b) par voie parentérale sous forme d'ampoules injectables contenant chacune jusqu'à 100 mg de principe actif (jusqu'à 8 ampoules par jour).

Revendications

1. N-oxydes de dérivés aminocycliques, caractérisés en ce qu'ils répondent à la formule générale :

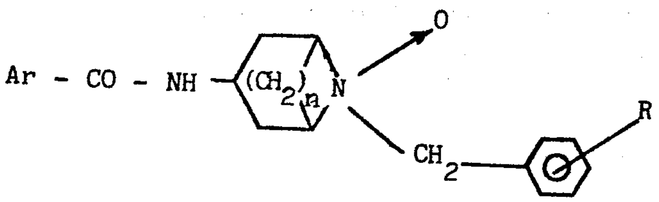

(I)

dans laquelle R représente un atome d'hydrogène, un ou deux atomes d'halogène ou un groupe alkyle en $C_1$-$C_4$, alkyloxy en $C_1$-$C_4$, cyano ou trifluorométhyle, n prend la valeur 2 ou 3 et Ar représente :

a) un groupe pyrimidinique de formule :

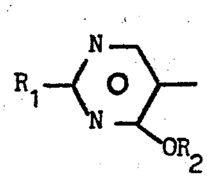

où $R_1$ = H, alkyle en $C_1$-$C_4$, alkoxy en $C_1$-$C_4$, $NH_2$, NH-alkyl en $C_1$-$C_4$, N(alkyl en $C_1$-$C_4)_2$, pyrrolidino, pipéridino ou morpholino et $R_2$ = alkyle en $C_1$-$C_4$ ;

b) un groupe aromatique de formule :

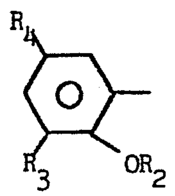

ou

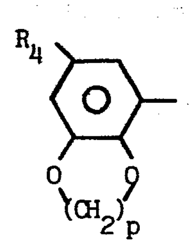

où p = 1 ou 2,

$R_2$ a les mêmes significations que précédemment,

$R_3$ = OH, alkyloxy en $C_1$-$C_4$ ou alkyle en $C_1$-$C_4$ et

$R_4$ = halogène, OH, alkyloxy en $C_1$-$C_4$, alkylsulfonyle en $C_1$-$C_4$,

$NO_2$, CN, CHO, (alkyl en $C_1$-$C_4$) carbonyle, $NH_2$, NH-alkyl en $C_1$-$C_4$, N(alkyl en $C_1$-$C_4$)$_2$, pyrrolidino, morpholino, pipéridino, $SO_2NH_2$, $SO_2$ N(alkyl en $C_1$-$C_4$)$_2$, pyrrolidinosulfonyle, pipéridinosulfonyle ou morpholinosulfonyle,

$R_3$ pouvant en outre représenter un groupe $NH_2$ ou NH-alkyl en $C_1$-$C_4$ quand $R_4$ = halogène et un atome d'hydrogène quand $R_4$ représente un groupe OH ou alkyloxy en $C_1$-$C_4$ ;

c) un groupe aromatique de structure :

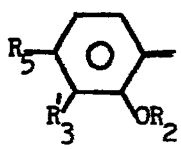

ou

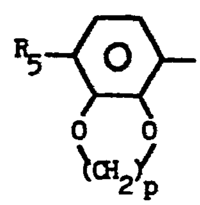

où $R_2$ et p ont les mêmes significations que précédemment, $R'_3$ = OH, alkyloxy en $C_1$-$C_4$ ou alkyle en $C_1$-$C_4$ et $R_5$ = $NH_2$, NH-alkyl en $C_1$-$C_4$, N(alkyl en $C_1$-$C_4$)$_2$, pyrrolidino, pipéridino, morpholino, halogène ou alkyloxy en $C_1$-$C_4$, $R'_3$ pouvant en outre représenter un atome d'hydrogène quand $R_5$ représente un groupe alkyloxy en $C_1$-$C_4$ ; ou

d) un groupe aromatique de structure :

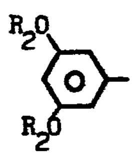

où $R_2$ a la même signification que précédemment ;

les composés de formule (I) possédant la forme axiale (Ia) :

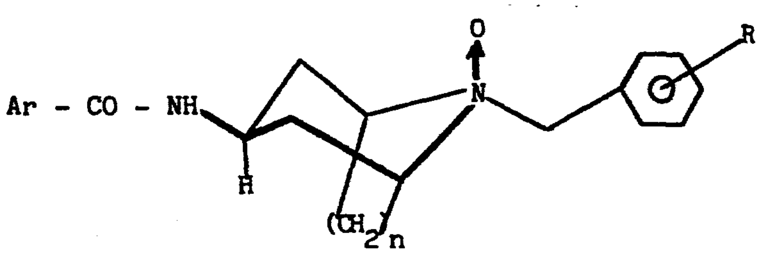

(Ia)

où n = 2 ou 3 et la chaîne ArCONH est en position équatoriale et l'atome d'oxygène du fragment 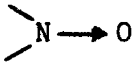 est en position axiale ou la forme équatoriale (Ib) :

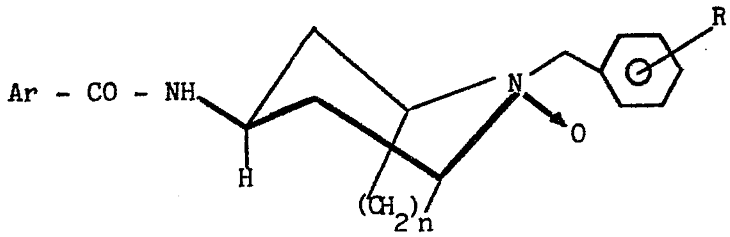

(Ib)

où n = 2 ou 3 et la chaîne ArCONH et l'atome d'oxygène du fragment 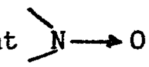 sont tous deux en position équatoriale ; ou étant constitués par un mélange de la forme axiale (Ia) et de la forme équatoriale (Ib).

2. Composés de structure (Ia) selon la revendication 1, pour lesquels l'ensemble (Ar-, R, n) prend l'une quelconque des significations suivantes :

( 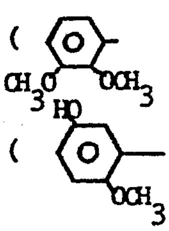 , H, 2), ( 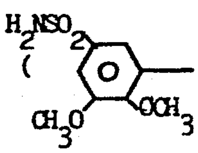 , H, 2), ( 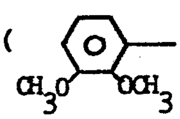 H, 3),
( , H, 2),

( 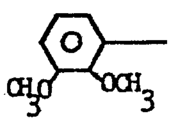 , 4-Br, 2), ( 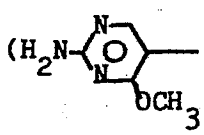 , 4 - $CH_3$, 2),

( 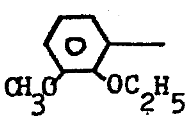 , H, 2).

3. Composés de structure (Ib) selon la revendication 1, pour lesquels l'ensemble (Ar-, R, n) prend l'une quelconque des significations suivantes :

( 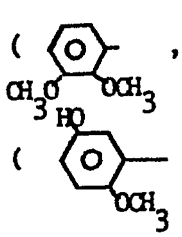 , H, 2), ( 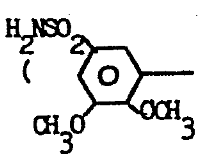 , H, 2), ( 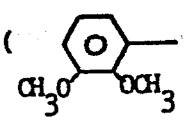 H, 3),
( , H, 2),

( 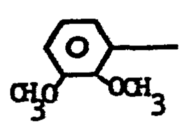 , 4-Br, 2), (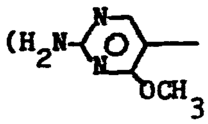 , 4 - $CH_3$, 2),

( 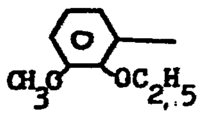 , H, 2).

4. Médicaments ayant notamment une activité sur le système nerveux central, caractérisés en ce qu'ils sont constitués par les composés selon l'une quelconque des revendications précédentes.

5. Composition pharmaceutique, caractérisée en ce qu'elle comprend à titre de principe actif au moins l'un des médicaments selon la revendication 4 en association avec un véhicule pharmaceutiquement acceptable.

6. Procédé de préparation des composés selon la revendication 1 de formule (I), caractérisé en ce qu'il consiste en une oxydation des composés de formule :

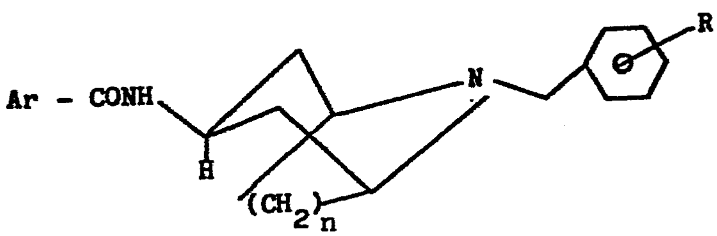

(II)

dans laquelle la chaîne ArCONH est en position équatoriale, n = 2 ou 3 et R et Ar ont la même signification que dans la revendication1, éventuellement suivie de la séparation des formes (Ia) et (Ib).

7. Procédé selon la revendication 6, caractérisé en ce que l'on réalise l'oxydation par mise en oeuvre d'un peracide.

8. Procédé selon la revendication 7, caractérisé en ce que le peracide est l'acide métachloroperbenzoïque.

9. Procédé selon la revendication 6, caractérisé en ce que l'on réalise l'oxydation par mise en oeuvre d'un peroxyde.

10. Procédé selon la revendication 9, caractérisé en ce que le peroxyde est l'eau oxygénée.

11. Procédé selon l'une quelconque des revendications 6 à 10, caractérisé en ce que la séparation des formes (Ia) et (Ib) est réalisée par chromatographie sur colonne.

12. Procédé selon la revendication 11, caractérisé en ce que la chromatographie est du type chromatographie liquide sur colonne de silice à haute ou moyenne pression.